**Europäisches Patentamt**

**European Patent Office**

**Office européen des brevets**

(19)

(11) Publication number: **0 311 616 B1**

# EUROPEAN PATENT SPECIFICATION

(12)

(45) Date of publication of patent specification :
25.09.91 Bulletin 91/39

(51) Int. Cl.⁵ : **A61K 37/66**

(21) Application number : 87903945.1

(22) Date of filing : 01.06.87

(86) International application number :
PCT/US87/01228

(87) International publication number :
**WO 87/07842 30.12.87 Gazette 87/29**

(54) **COMBINATIONS OF GAMMA INTERFERONS AND ANTI-INFLAMMATORY OR ANTI-PYRETIC AGENTS FOR TREATING DISEASES.**

The file contains technical information
submitted after the application was filed and
not included in this specification

(30) Priority : 17.06.86 US 875401

(43) Date of publication of application :
19.04.89 Bulletin 89/16

(45) Publication of the grant of the patent :
25.09.91 Bulletin 91/39

(84) Designated Contracting States :
AT BE CH DE FR GB IT LI LU NL SE

(56) References cited :
EP-A- 0 177 342
EP-A- 0 177 910
DE-A- 3 436 638
US-A- 4 246 263
US-A- 4 252 797
US-A- 4 390 545

(56) References cited :
EXPERIENTIA, vol. 42, 1986, pages 177-179,
Birkhäuser Verlag, Basel, CH; R. CAVALLO et
al.: "Cortisol and immune interferon can in-
teract in the modulation of human natural
killer cell activity"
Chemical Abstracts, Volume 104, No. 17, is-
sued 28 April 1986 (Co- lumbus, Ohio, USA),
Cavalllo, "Cortisol and Immune Interferon
Can Interact in the Modulation of Human
Natural Killer Cell Activity". See page 527,
Column 1, the abstract No. 146904k, Experien-
tia, 1986, 42(2), 177-9 (Eng.).

(73) Proprietor : BIOGEN, INC.
14 Cambridge Center
Cambridge Massachusetts 02142 (US)

(72) Inventor : Reich, Steven D.
32 Overlook Drive
Southborough, MA 01772 (US)

(74) Representative : Bannerman, David Gardner et
al
Withers & Rogers 4 Dyer's Buildings Holborn
London, EC1N 2JT (GB)

## Description

This invention relates to combinations useful for the treatment of malignant diseases, non-malignant diseases, rheumatic diseases and allergies. More particularly, this invention relates to combinations of gamma interferons and anti-inflammatory or anti-pyretic agents for treating rheumatic diseases, such as rheumatoid arthritis, malignant diseases, such as melanomas, non-malignant diseases, such as common or anogenital warts, and allergic diseases, such as asthma. According to this invention, natural or recombinant gamma interferons are used in combination with anti-inflammatory agents or anti-pyretic agents in order to enhance the efficacy of gamma interferon in the treatment of these diseases.

Malignant diseases are a group of diseases characterised by tumorigenic or neoplastic cell growth. Such diseases include malignant hematological systemic diseases, carcinomas, sarcomas, myelomas, melanomas, lymphomas and papillomas. Non-malignant diseases, including non-malignant tumors, are also characterised by neoplastic cell growth which is localized to a specific area. The transformation of normal cells within the body into either malignant or non-malignant neoplasms may be induced by viral infections, chemical carcinogens, radiation, physical agents or spontaneous tumorigenic growth. Common and anogenital warts, for example, are benign growths which are induced by papilloma viruses.

The precise etiology of many malignant and non-malignant diseases remains unknown. Accordingly, treatments for these diseases are limited, and effective agents are not always conventionally available for a specific disease. Such diseases have been treated, for example, by surgical techniques or by non-surgical methods including chemotherapy, radiation and immunotherapy. Viral warts have also been treated by cryotherapy, salicylic acid preparations of formalin preparations. Any value of such treatment techniques, however, is often diminished by adverse side effects or risks attendant with their use. For example, non-surgical techniques having immunosup- pressant effects may increase the patient's susceptibility to secondary infections. Surgical treatments to excise malignant or non-malignant tumors involve risks which accompany any invasive procedure and may not effectively remove or eliminate the entire tumor or malignant cell population. Moreover, certain malignant diseases are resistant to conventional treatment techniques. For example, most skin melanomas are considered to be radioresistant. No single agent or combination chemotherapy has been successful in effecting consistent regressions of malignant melanomas. Malignant renal cell carcinoma is also resistant to available single agent and combination chemotherapies.

Typically, allergic diseases are treated by a variety of drugs aimed at counteracting the symptoms of the allergic reaction. However, these prior treatments may be useful only on a short term basis and often have adverse or unwanted side effects. For example, corticosteroids may be used to treat severe allergic reactions such as severe chronic bronchial asthma. Several toxic effect may result from withdrawal from or continued use of large doses of corticosteroids. For example, prolonged therapy may result in suppression of pituitary-adrenal function, fluid and electrolyte disturbances, hyperglycemia and glycosuria, peptic ulcers, osteoporosis, myopathy and behavioral disturbances. Corticosteroids also immunosuppress the patient and thereby increase his susceptibility to infectious disease. [A.G. Gilman et al. (Eds.), The Pharma- cological Basis of Therapeutics, pp. 1482-90 (1980).]

Rheumatic diseases are a group of diseases that affect the musculo-skeletal and connective tissues of the body. Such diseases are characterised by chronic inflammation which often leads to permanent tissue damage, deformity, atrophy and disability. Rheumatoid arthritis, a progressive rheumatic disease, affects as many as 1-2% of all adults [P.D. Utsinger et al., Rheumatoid Arthritis, p. 140 (1985)]. Juvenile chronic arthritis, which occurs in children and adolescents, differs from rheumatoid arthritis as seen in adults and can lead to impaired development and growth [B.Z.P. Anzell, "Juvenile Chronic Polyarthritis", Arthr. Rheum. Supp., 20, pp. 176-80 (1977)].

Most rheumatic diseases are thought to be autoimmune diseases caused by a patient's altered immune response to an as yet unidentified antigen or antigens [H.O. McDevitt and B. Benacerraf, "Genetic Control Of Specific Immune Responses", Adv. Immunol., 11, p. 31 (1969)]. Upon serological examination, the majority of patients diagnosed with clinical rheumatic disease test seropositive for rheumatoid factor, an abnormal gamma-globulin [R.B. Berkow et al. (Eds.), The Merck Manual (1982)].

The etiology of the majority of rheumatic diseases remains unknown. Treatments for these diseases are limited, and effective agents are not conventionally available for a specific disease [K. Kruger, "Neue Therapieprinzipien In Der Rheumatologie" ("New Therapeutic Principles In Rheumatology"), Munch. Med. Wschr., 126, pp. 1084-86 (1984)].

Therapeutics used in the treatment of rheumatic diseases and certain tumorigenic diseases fall into two classes. The first class of therapeutics - - symptomatics - - act on the symptoms of the disease, exerting effects both upon and throughout the total course of administration. Such symptomatics include salicylates, glucocorticoids and non-steroidal anti-inflammatory agents, such as indomethacin. Symptomatics are also used in the treatment of allergic diseases.

The symptomatic therapeutics act on symptoms of inflammation which are characteristic in patients with certain malignant, tumorigenic and rheumatic diseases. Rheumatic diseases and certain spontaneous human tumors are accompanied by increased concentrations of prostaglandins in the body [A.G. Gilman et al. (Eds.), The Pharmacological Basis of Therapeutics, p. 678 (1980)]. Increased levels of prostaglandins released into the body are thought to be responsble for the symptoms of the inflammatory process [id., p. 677]. It has been demonstrated that glucocorticoids, salicylates and other non-steroidal anti-inflammatory agents inhibit prostaglandin biosynthesis [Id., pp. 684-85, 1480]. The inhibition of prostaglandin synthesis appears to be a major factor in the therapeutic activity of these anti-inflammatory agents.

The second class of therapeutics act on the pathogenesis of the disease, exerting their effects only after the initial weeks of administration, yet having effects lasting beyond the cessation of treatment. Such therapeutics include cortisones, immunosuppressants, and, for the treatment of rheumatic diseases, gold compounds, D-penicillamine and chloroquine.

These conventional therapeutic agents typically must be administered over long periods of time and any therapeutic value is often diminished by adverse side effects or serious toxicity. For example, immunosuppressants may increase the patient's susceptibility to infection. And, therapies based on gold compounds, penicillamine or chloroquine may exert toxic effects on the patient's system.

Alpha interferon has been reported to be ineffective in the treatment of rheumatoid arthritis [A. Kajender et al., "Interferon Treatment Of Rheumatoid Arthritis", Lancet, i, pp. 984-85 (1979)]. This lack of efficacy may not be surprising because certain rheumatic diseases, such as rheumatoid arthritis, are characterized by an endogenous formation of interferons to which no therapeutic activity has been ascribed. Furthermore, in some cases, high circulating levels of interferons have been suggested without real clinical data as a factor contributing to the pathogenesis of rheumatic diseases [J.J. Hooks et al., "Immune Interferon In The Circulation Of Patients With Autoimmune Disease", N. Engl. J. Med., 301, pp. 5-8 (1979) ; O.T. Preble et al., "Systemic Lupus Erythematosus : Presence In Human Serum Of An Unusual Acid-Labile Leukocyte Interferon", Science, 126, pp. 329-31 (1982) ; M. Degré et al., "Immune Interferon In Serum And Synovial Fluid In Rheumatoid Arthritis And Related Disorders", Ann. Rheumatic Dis., 42, pp. 672-76 (1973) ; A. M. Arvin and J.J. Miller, "Acid Labile α-Interferon In Sera And Synovial Fluids From Patients With Juvenile Arthritis", Arthritis and Rheumatism, 27, pp. 582-85 (1984) ; T.O. Rosenbach et al., "Interferon Triggers Experimental Synovitis And May Potentiate Auto-Immune Disease In Humans", Clin. Rheumatol., 3, pp. 361-64 (1984)]. It has been reported in an in vitro study that gamma interferon increases the proliferation of the human synovial fibroblasts involved in the rheumatoid arthritis disease state and that this increased proliferation is further enhanced by the addition of prednisone - - a steroidal anti-inflammatory agent [C.E. Brinckerhoff and P.M. Guyre, "Increased Proliferation of Human Synovial Fibroblasts Treated With Recombinant Immune Interferon," J. Immunol., 134, pp. 3142-46 (1985)].

Interferons such as alpha interferon, beta interferon, and gamma interferon have been used as therapeutic agents for malignant diseases or non-malignant tumors in varying dosages and via several modes of administration. Generally, low dose regimens of up to about 3 million I.U. of alpha or beta interferons have been reported to be ineffective for the systemic treatment of tumors or inferior to higher doses in those treatments. See, for example, J.M. Kirkwood and M.S. Ernstoff, "Interferons In The Treatment Of Human Cancer", J. Clin. Oncol., 2, pp. 336-52 (1984) ; A. Billiau, Contr. Oncol., 20, pp. 251-69 (1984) ; E.M. Bonnem and R.J. Spiegel, Interferon-α : Current Status And Future Promise", J. Biol Resp. Modif., 3, pp. 580-98 (1984) ; R. L. Knobler et al., Neurology, 34, pp. 1273-79 (1984) ; M.A. Faerkkilae et al., Act. Neur. Sci., 69, pp. 184-85 (1985) ; S.B. Greenberg and M.W. Harmon, J.A. Armstrong in P.E. Came and W.A. Carter : Interferons and Their Applications, Springer Verlag (1984). Only upon local treatment (intratumoral, peritumoral, intraventricular, intrathecal, intralesional, perilesional or intranasal application) have doses of less than 3 million I.U. been reported to demonstrate activity comparable to that effected by systemic application of higher doses.

Gamma interferon has been shown in in vitro and in vivo studies to be a potent anti-tumor agent and regulator of the immune system (R.D. Williams and L. Nunes, "Effect of Gamma Interferon on Human Cancer Cells in Vitro and in Vivo in Nude Mice", J. of Urology, 133 (1985) ; Vilcek et al., "Interferon-Gamma : Interactions with Other Lymphokines," Interferon Syst. Proc. Int. TNO Meeting 2nd (1983)]. The antiviral activity of gamma interferon has been reported to be suppressed by certain non-steroidal anti-inflammatory agents - - oxyphenylbutazone, aspirin, indomethacin, naproxen - - which are inhibitors of prostaglandin synthetase (fatty acid cyclo-oxygenase) [R. Pottathil et al., "Establishment of the Interferon-mediated Antiviral State : Role of Fatty Acid Cyclooxygenase", Proc. Natl. Acad. Sci. USA, 77, pp. 5437-40 (1980)]. It has been suggested that prostaglandin synthetase function, which is necessary for prostaglandin biosynthesis, may be essential for complete expression of the interferon-mediated anticellular and anti-viral states [Pottathil et al., supra ; K.A. Chandrabose et al., "Interferon-resistant Cell Line Lacks Fatty Acid Cyclooxygenase Activity," Science, 212, pp. 329-31

3

(1981)].

Phase I studies carried out on patients with tumors indicate that the toxicity profile of IFN-γ is similar to those found in other interferons (S. Yamazaki, Jpn. J. Med. Sci. Biol., 37, pp. 209-23 (1984) ; S.A. Sherwin et al., "A Preliminary Phase I Trial Of Partially Purified Interferon-γ In Patients With Cancer", J. Biol. Resp. Modif., 3, pp. 599-607 (1984) ; J.U. Guttermann et al., "Pharmacokinetic Study Of Partially Pure γ-Interferon In Cancer", Cancer Res., 44. pp. 4164-71 (1984) ; N- Niederle et al., in H. Kirchner and H. Schellekens (Eds,), The Biology of the Interferon System 1984 (1985) ; R. Kurzrock et al., "Pharmacokinetics, Single-Dose Tolerance, and Biological Activity of Recombinant γ-Interferon in Cancer Patients," Cancer Research, 45 pp. 2866-72 (1985); M. van der Burg, M. Edelstein, L. Gerlis, C-M. Liang, M. Hirschi, A. Dawson, "Recombinant Interferon-Gamma (Immuneron®) : Results Of A Phase I Trial In patients With Cancer", J. Bio. Resp. Modif., 4, pp. 264-72 (1985)].

Although IFN-γ has shown promise as a potent antitumor agent and regulator of the immune system, the use of IFN-γ in treating rheumatic, malignant and non-malignant diseases has been limited. Constitutional signs, such as fever, headache and flu-like symptoms generally represent the dose-limiting toxicity of IFN-γ. A dosage schedule of 3 mg/m²/day IFN-γ over a 14 day period appears to be at or above the maximally tolerated dose [J.M. Kirkwood, "Phase I/II Study Of Recombinant Interferon Gamma (IMMUNERON®) Administered By 2 Or 24 Hour Infusion In 30 Patients With Melanoma", Abstract-Cytokine Research : Gamma Interferon, 3rd European Conference on Clinical Oncology, Stockholm, June 18, 1985]. Such constitutional side effects of IFN-γ may limit dosing of IFN-γ in individuals afflicted with rheumatic, malignant or non-malignant diseases.

To date therefore, conventional methods and therapeutic agents have not proved to be effective in the treatment of many malignant, non-malignant, allergic and rheumatic diseases. Accordingly, the need exists for a therapeutic method which avoids the disadvantages of these conventional methods and agents while providing effective treatment for these diseases.

The present invention solves the problems referred to above by providing effective combinations and methods for the treatment of malignant diseases, non-malignant diseases, allergic diseases and rheumatic diseases. According to this invention, natural or recombinant gamma interferons are used in combination with steroidal or non-steroidal anti-inflammatory agents or with anti-pyretic agents for treating rheumatic diseases, malignant diseases, non-malignant diseases and allergies. Advantageously, the combinations and methods of this invention increase the effectiveness of IFN-γ in the treatment of these diseases. In addition, the use of the combinations and methods of this invention may reduce the level and duration of treatment which would be required by therapies based on the use of anti-inflammatory or anti-pyretic agents alone.

## BRIEF DESCRIPTION OF THE DRAWINGS

Figure 1 is a graphical representation of the effect of treatment with one of two non-steroidal anti-inflammatory agents, indomethacin or naproxen, alone or in combination with gamma interferon, on the growth of BL/MEX6 human melanoma in a 6-day Subrenal Capsule™ Assay.

Figure 2 is a graphical representation of the effect of treatment with an anti-inflammatory agent - - acetyl salicylic acid, or an anti-pyretic agent - - acetaminophen, alone or in combination with gamma interferon, on the growth of BL/MEX6 human melanoma in a 6-day Subrenal Capsule™ Assay.

Figure 3 is a graphical representation of the effect of treatment with one of two steroidal anti-inflammatory agents, cortisone or prednisone, alone or in combination with gamma interferon, on the growth of BL/MEX6 human melanoma in a 6-day Subrenal Capsule™ Assay.

In order that the invention herein described may be more fully understood, the following detailed description is set forth.

In the description, the following terms are employed :

Gamma interferon or IFN-γ - - In accordance with the interferon nomenclature announced in Nature, 286, p. 110 (1980) and recommended by the Interferon Nomenclature Committee in Archives of Virology, 77, pp. 283-85 (1983). IFN-γ was originally referred to as "immune interferon".

IFN-γ is a lymphokine which is naturally produced in minute quantities together with other lymphokines by lymphocytes. It is primarily produced by T-lymphocytes, spontaneously or in response to various inducers such as mitogens, specific antigens or specific antibodies [W.E. Stewart, II, The Interferon System, pp. 148-49 (1981)]. IFN-γ is a glycoprotein having a molecular weight between 10,000 and 25,000 (or 17,000 in non-glycosylated form). IFN-γ has also been cloned and expressed in various host-vector systems. The nucleotide sequence of cloned IFN-γ indicates that it is composed of 143-46 amino acids.

As used in this application and claims, "IFN-γ" includes all proteins, polypeptides and peptides which are natural or recombinant IFN-γs, or derivatives thereof, and which are characterized by the biological activity of those IFN-γs against rheumatic, allergic, malignant or non-malignant diseases. These include IFN-γ-like compounds from a variety of sources such as natural IFN-γs, recombinant IFN-γs and synthetic or semi-synthetic IFN-γs.

Malignant Disease - - Any disease characterized by tumorigenic or neoplastic cell growth, including malignant hematological systemic diseases, carcinomas, sarcomas, myelomas, melanomas, leukemias, lymphomas and papillomas.

Non-malignant Disease -- Any disease characterized by an undesirable proliferation of cells which is localized to the site of origin, including common or anogenital warts and any other benign growth.

Rheumatic Disease - - Any disease which is (i) characterized by inflammation or degeneration of musculo-skeletal or connective tissue structures of the body, particularly the joints, spinal cord, soft tissue or bone, and including muscles, tendons, cartilage, synovia and fibrous tissues, (ii) accompanied by pain, stiffness or impairment of locomotion or function of those structures and, in some cases, (iii) often accompanied by serological evidence of rheumatoid factor.

Rheumatic diseases include, but are not limited to, inflammatory rheumatism, degenerative rheumatism, extra-articular rheumatism and collagen diseases. Examples of such diseases are systemic lupus erythematosus, rheumatoid arthritis or chronic polyarthritis, progressive systemic scleroderma, dermatomyositis, psoriasis arthropathica, muscular rheumatism, periarthritis humeroscapularis, panarthritis nodosa, myositis, myogelosis, arthritis uratica, chondrocalcinosis and juvenile chronic arthritis ("Stills disease").

This invention relates to combinations useful for treating rheumatic diseases, malignant diseases, non-malignant diseases and allergies.

The IFN-γs which are useful in the combinations of this invention may be produced by purification from natural sources using conventional techniques or by recombinant techniques.

For example, some established or transformed cell lines produce natural IFN-γ constitutively in vitro [N. Fujii et al., "Spontaneous Production Of γ-Interferon In Cultures Of T Lymphocytes Obtained From Patients With Behcet's Disease", J. Immunol., 130, pp. 1683-86 (1983)]. A T cell hybridoma variant clone was also found to produce IFN-γ, together with other lymphokines, in response to stimulation with concanavalin A [A. Zlotnick et al., "Coordinate Production By A T Cell Hybridoma Of Gamma Interferon And Three Other Lymphokine Activities : Multiple Activities Of A Single Lymphokine ?", J. Immunol., 131, pp. 794-80 (1983)].

Also among the IFN-γs useful in the combinations of this invention are the IFN-γs produced in vitro by a variety of cells in response to various interferon inducers. For example, these IFN-γs include IFN-γs produced by human buffy-coat leukocytes after exposure to phytohemagglutinin P, concanavalin A and staphylococcal enterotoxin A ("SEA"), M. DeLay et al., "Interferon Induced In Human Leukocytes By Mitogens :

Production, Partial Purification and Characterization", Eur. J. Immunol., 10, pp. 877-83 (1980) ; in human splenocytes after stimulation with SEA, R. Devos et al., "Interferon Induced In Human Leukocytes By Mitogens: Production, Partial Purification and Characterization", Eur. J. Immunol., 10, pp. 877-83 (1980) ; in human splenocytes after stimulation with SEA, R. Devos et al., "Isolation And Characterization of IFN-Gamma mRNA Derived From Mitogen-Induced Human Splenocytes", J. Interferon Res., 2, pp. 409-20 (1982) ;

by an IL-2-independent murine T cell line after stimulation by phorbol 12-myristate 13-acetate, W.R. Benjamin et al., "Production of Immune Interferon by an Interleukin 2-Independent Murine T cell Line", Proc. Natl. Acad. Sci. USA, 79, pp. 5379-83 (1982) ; in lymphoid cells by using calcium ionophore A-23187, F. Dianzani et al., "Human Immune Interferon : Induction in Lymphoid Cells by a Calcium Ionophore", Infection and Immunity, 29, pp. 561-63 (1980) ; and in thymocytes, G.H. Reem et al., "Gamma Interferon Induction In Human Thymocytes Activated By Lectins and B Cell Lines", Infection and Immunity, 37, pp. 216-21 (1982). See also United States patents 4,376,821 and 4,376,822 and European patent application 63,482.

These natural IFN-γs have been subsequently purified to some extent and partially characterized. See, for example, United States patents 4,289,690, 4,314,935 and 4,382,027, European patent application 87,686, O'Malley, "Affinity Chromatography of Human Immune Interferon", Methods in Enzymology, 78, pp. 540-45 (1981), and Y.K. Yip et al., "Partial Purification and Characterization of Human γ (Immune) Interferon", Proc. Natl. Acad. Sci. USA, 78, pp. 1601-05 (1981).

IFN-γs useful in the combinations of this invention may also be produced in large amounts using recombinant DNA technology. See, e.g., European patent application 88,540 ; R. Derynck et al., "Human Interferon γ Is Encoded By A Single Class Of mRNA", Nucleic Acids Research, 10, pp. 3605-13 (1982) ; R. Derynck et al., "Expression Of The Human Interferon-γ cDNA In Yeast", Nucleic Acids Res., 11, pp. 1819-37 (1983) ; R. Devos et al., "In Vitro Translation And Characterization Of Human IFN-γ mRNA", J. Clin. Hemator. Oncol., 11(4), p. 114 (1981) ; and R. Devos et al., "Molecular Cloning Of Human Immune Interferon cDNA And its Expression in Eukaryotic Cells", Nucleic Acids Research, 10(8), pp. 2487-501 (1982).

The anti-inflammatory agents useful in the combinations of this invention include steroidal and non-steroidal anti-inflammatory agents. Steroidal anti-inflammatory agents include, but are not limited to, cortisol, prednisone, prednisolone, 6 α-methylprednisolone, cortisone, triamcinolone, paramethasone, betamethasone, dexamethasone and other compounds having similar anti-inflammatory activity. Non-steroidal anti-inflammatory agents include, but are not limited to, acetyl salicylic acid (aspirin), methyl salicylate, sodium salicylate, phenylbutazone, oxyphenbutazone, antipyrine, aminopyrine, dipyrone, apazone, indomethacin, sulindac, mefenamic acid, tolmetin sodium, ibuprofen, naproxen, fenoprofen, flurbiprofen, ketoprofen and other compounds having similar anti-inflammatory activity. Other anti-inflammatory agents useful in the combinations of this invention are lipocortins derived from natural sources or lipocortins and lipocortin-like polypeptides produced by recombinant techniques [See United States patent application serial nos. 690,146 ; 712,376 ; 765,877 ; and 772,892 ; B. Wallner et al., "Cloning And Expression Of Human Lipocortin A Phospholipase A-2 Inhibitor With Potential Anti-Inflammatory Activity", Nature, 320, pp. 77-81 (1986)].

Anti-pyretic agents useful in the combinations of this invention include, but are not limited to acetyl salicylic acid, methylsalicylate, sodium salicylate, phenacetin, acetaminophen, indomethacin, sulindac, mefenamic acid, tolmetin sodium, ibuprofen, naproxen, fenoprofen, flurbiprofen, ketoprofen and other compounds having similar anti-pyretic activity.

The combinations of this invention may be used to treat any mammal, including humans. Mammals can be treated by the pharmaceutically acceptable administration of IFN-$\gamma$, in combination with an anti-inflammatory agent or an anti-pyretic agent, in pharmaceutically effective dosages and for a period of time sufficient to exert immunologic effects, reduce the symptoms of the rheumatic or allergic disease or prevent their recurrence or, alternatively, to inhibit malignant or undesirable non-malignant cell proliferation.

The use of the compositions of this invention advantageously enhances the immunological activity of IFN-$\gamma$ in the treatment of rheumatic or allergic diseases. Alternatively, these compositions and processes enhance the inhibitory action of IFN-$\gamma$ against malignant or undesirable non-malignant cell proliferation. In addition, the use of IFN-$\gamma$ in combination with an anti-inflammatory or anti-pyretic agent may reduce the duration and level of treatment which would be required by therapies based upon an anti-inflammatory agent or an anti-pyretic agent alone.

According to this invention, IFN-$\gamma$ may be prepared for administration to the patient in any pharmaceutically acceptable dosage form including those which may be administered to a patient intravenously as bolus or by continuous infusion over a period of hours, days, weeks or months, intramuscularly - - including paravertebrally and periarticularly - - subcutaneously, intracutaneously, intra-articularly, intrasynovially, intrathecally, intralesionally, periostally, or by oral or topical routes.

Such dosage forms may include pharmaceutically acceptable carriers and adjuvants which are known to those of skill in the art. These carriers and adjuvants include, for example, ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes such as protamine sulfate, disodium hydrogen phosphate, potassium hydrogen phosphate, sodium chloride, zinc salts, colloidal silica, magnesium trisilicate, polyvinyl pyrrolidone, cellulose-based substances and polyethylene glycol. Adjuvants for topical or gel base forms of IFN-$\gamma$ may be selected from the group consisting of sodium carboxymethylcellulose, polyacrylates, polyoxyethylenepolyoxypropylene-block polymers, polyethylene glycol and wood wax alcohols. For all administrations, conventional depot forms may be used. Such forms include, for example, nano-capsules, micro-capsules, liposomes, plasters and biodegradable polymers.

The most effective mode of administration and dosage regimen of the IFN-$\gamma$ preparation will depend upon the type of disease to be treated, the severity and course of that disease, previous therapy, the patient's health status and response to IFN-$\gamma$ and the judgment of the treating physician. IFN-$\gamma$ may be administered to the patient at one time or over a series of treatments. Preferably, the anti-inflammatory agent or anti-pyretic agent and the IFN-$\gamma$ are administered concurrently to the patient. Concurrent administration involves treatment with the anti-inflammatory agent or anti-pyretic agent at least on the same day (within 24 hours) of treatment with IFN-$\gamma$ and may involve continued treatment with the anti-inflammatory agent or anti-pyretic agent on days that the IFN-$\gamma$ is not administered. Conventional modes of administration and standard dosage regimens of anti-inflammatory agents or anti-pyretic agents may be used. [See A.G. Gilman et al. (Eds.), The Pharmacological Basis of Therapeutics, pp. 697-713, 1482, 1489-91 (1980) ; and P.D. Utsinger et al., Rheumatoid Arthritis, pp. 555-56, 593 (1985).]

In the treatment of rheumatic diseases, allergic diseases or non-malignant diseases, IFN-$\gamma$ may be administered subcutaneously in an initial dose of between about 10 µg/day and 500 µg/day, adjusted in the individual patient to eliminate side effects and continued until symptoms have been alleviated to the desired level. Preferably, IFN-$\gamma$ may be administered at a dose of 100 µg/day. Alternatively, in the treatment of non-malignant diseases such as common or anogenital warts, IFN-$\gamma$ may be administered intralesionally.

Once improvement in the patient's condition has occurred, a maintenance dose of between about 10 µg/day and 500 µg/day of IFN-$\gamma$ may be administered two to five times per week. Subsequently, the dosage and/or frequency of administrations may be reduced, as a function of the symptoms, to a level at which the improved condition is retained.

Typically, treatment with one of a group of anti-inflammatory or anti-pyretic agents proceeds concurrently with the administration of IFN-γ. For example, oral administration of prednisone, a steroidal anti-inflammatory agent, may begin with about 60-100 mg/day, gradually tapered down to a dose of about 5-10 mg/day. Alternatively, the equivalent dose of another steroidal anti-inflammatory agent may be substituted (e.g., a final low daily dosage of about .75-1.5 mg dexamethasone or about 5-10 mg prednisolone). If non-steroidal anti-inflammatory agents or anti-pyretic agents are used, dosages are titrated to the individual patient. For example, aspirin (acetyl salicylic acid) may be administered orally at doses slightly below those which produce adverse side effects (generally about 5-6 g daily). Alternatively, acetaminophen (paracetamol) may be administered orally at a dosage of about 300-975 mg every 4 hours. Indomethacin (about 25 mg 3 times per day orally), tolmetin (about 600-800 mg/day) or ibuprofen (about 1200-1600 mg/day), or therapeutic doses of other non-steroidal anti-inflammatory agents or anti-pyretic agents may also be used. Other dosage regimens of IFN-γ and anti-inflammatory or anti-pyretic agents are also useful.

In the treatment of malignant diseases, IFN-γ may be administered intravenously at an initial dosage of between about 100 μg/m²/day and 5 mg/m²/day two to seven times per week. The dosage of IFN-γ is adjusted according to the tolerance of the individual patient and may be reduced to between about 100 and 125 μg/m²/day seven or fewer times per week. An anti-inflammatory agent or an anti-pyretic agent may be administered concurrently with the IFN-γ treatment using the dosage regimens described above. Other dosage regimens of IFN-γ and anti-inflammatory or anti-pyretic agents are also useful. Treatment with IFN-γ and an anti-inflammatory agent or anti-pyretic agent may continue until a desired suppression of disease symptoms is observed.

Once improvement in the patient's condition has occurred, a maintenance dose of between about 100 μg/m²/day and 5 mg/m²/day of IFN-γ is administered two to five times per week. Subsequently, the dosage and/or frequency of administrations may be reduced, as a function of the symptoms, to a level at which the improved condition is retained. Typically, such a maintenance schedule may involve the administration of between about 100 μg/m²/day and 5 mg/m²/day of IFN-γ to the patient two to five times per week on alternate weeks. When the symptoms have been alleviated to the desired level, treatment should cease. Patients may, however, require intermittent treatment on a long term basis upon recurrence of disease symptoms.

In order that the invention described herein may be more fully understood, the following examples are set forth. It should be understood that these examples are for illustrative purposes only and are not to be construed as limiting this invention in any manner.

These examples represent the _in vivo_ effects of anti-inflammatory and anti-pyretic agents alone, and in combination with IFN-γ, in inhibiting tumor growth. These examples demonstrate that combinations of IFN-γ with anti-inflammatory agents or anti-pyretic agents were effective in inhibiting tumor growth to a greater degree than treatments with any one of IFN-γ, anti-inflammatory agents or anti-pyretic agents alone. The stability of body weights after treatment with the combinations reflects the lack of systemic toxicity of IFN-γ, anti-inflammatory agents or anti-pyretic agents.

In each example, following the standardized protocol for the Subrenal Capsule™ Assay [A.E. Bogden, P.M. Haskell, D.J. LePage, D.E. Kelton, W.R. Cobb, H.J. Esber, "Growth Of Human Tumor Xenografts Implanted Under The Renal Capsule Of Normal Immunocompetent Mice", Exp. Cell Biology, 47, pp. 281-93 (1979)], we implanted BL/MEX6 human melanoma established in serial transplantation (Bogden Laboratories) under the renal capsule of normal, immunocompetent mice (BALB/c × DBA/2) F₁ (CDF₁) (Examples 1 and 2) and into (B6C3F₁) mice (Example 3). Each mouse weighed approximately 25 grams. We then divided the mice into treatment groups as follows : group 1 : 10 mice, groups 2-7 : 8 mice each.

Over a five-day period, we treated each of groups 4-7 of mice with one of the following anti-inflammatory agents or anti-pyretic agents, either singly or in combination with IFN-γ (IFN-γ dosage : 2,000 μg/Kg/inj., b.i.d., i.m.) :

| | | |
|---|---|---|
| Indomethacin | 4.0 mg/Kg/inj., b.i.d., p.o. | (Example 1) |
| Naproxen | 124.0 mg/Kg/inj., b.i.d., p.o. | (Example 1) |
| Acetyl salicylic acid | 328.0 mg/Kg/inj., b.i.d., p.o. | (Example 2) |
| Acetaminophen | 192.0 mg/Kg/inj., b.i.d., p.o. | (Example 2) |
| Cortisone | 20.0 mg/Kg/inj., b.i.d., s.c. | (Example 3) |
| Prednisone | 20.0 mg/Kg/inj., b.i.d., s.c. | (Example 3) |

The anti-inflammatory agents or anti-pyretic agents were administered prior to IFN-$\gamma$ in all combination treatment groups. In each example, we treated groups 1-3 of mice as follows :

Group 1 :    0.25 ml/inj., b.i.d., i.m. PBS (vehicle treated control)
Group 2 :    400 mg/Kg/inj., s.c. DTIC [dacarbazine] (positive chemotherapy control)
Group 3 :    2,000 $\mu$g/Kg/inj., b.i.d., i.m. IFN-$\gamma$

In each example, for each mouse, we recorded initial body weight, implanted a tumor and recorded an initial in situ tumor measurement (two diameters) in ocular micrometer units (omu). We administered treatment on days 1-5. Survivors were recorded and any mice dying prior to sacrifice were necropsied. On day 6, the mice were sacrificed, final body weights were recorded, implanted kidneys were removed and final in situ tumor measurements were made to determine tumor growth inhibition or regression. We calculated the ratio of average final body weight to initial body weight (FBW/IBW) to monitor systemic toxicity. Change in ($\Delta$) tumor size between day of sacrifice (day 6) and day of tumor implant (day 0) was calculated individually and for each group.

In these examples, the IFN-$\gamma$ used to inhibit tumor growth was recombinant human IFN-$\gamma$, supplied by Biogen Research Corp. (Cambridge, Mass.). The specific activity of Biogen's recombinant human IFN-$\gamma$ was in the range of 10-30 $\times$ $10^6$ I.U./mg of protein. The various anti-inflammatory and anti-pyretic agents were supplied by Sigma Chemical Company.

### Example 1

To demonstrate the effect of indomethacin and naproxen on antitumor activity of IFN-$\gamma$, we treated groups 1-7 of mice as follows :

## TABLE I

| Group # | Treatment |
|---|---|
| 1 | PBS, 0.25 ml/inj., QD1-5, b.i.d., i.m. (vehicle treated control) |
| 2 | DTIC, 400 mg/Kg/inj., QD1-5, s.c. (positive chemotherapy control) |
| 3 | IFN-$\gamma$, 2000 $\mu$g/Kg/inj., QD1-5, b.i.d., i.m. |
| 4 | Indomethacin, 4.0 mg/Kg/inj., QD1-5, b.i.d., p.o. |
| 5 | Naproxen, 124.0 mg/Kg/inj., QD1-5, b.i.d., p.o. |
| 6 | Indomethacin, 4.0 mg/Kg/inj., QD1-5, b.i.d., p.o.; IFN-$\gamma$, 2000 $\mu$g/Kg/inj., QD1-5, b.i.d., i.m. |
| 7 | Naproxen, 124.0 mg/Kg/inj., QD1-5, b.i.d., p.o.; IFN-$\gamma$, 2000 $\mu$g/Kg/inj., QD1-5, b.i.d., i.m. |

The results obtained are set forth in Table II :

## TABLE II

### RESPONSE OF BL/MEX6 HUMAN MELANOMA TO INDOMETHACIN, NAPROXEN AND GAMMA INTERFERON USING THE SIX DAY SUBRENAL CAPSULE[M] ASSAY

| Group No.[1] (No. Animals) | Avg. Δ Tumor Size[2] (mean ± S.D.) | Percent Test/Control | Test-Control[3] | FBW/IBW[4] |
|---|---|---|---|---|
| 1 (10) | 3.7±1.95 | - | - | 0.98 |
| 2 (7) | 0.5±0.7*** | 14 | -3.20 | 0.86 |
| 3 (8) | 2.0±0.98* | 54 | -1.70 | 0.98 |
| 4 (8) | 3.1±1.69 | 84 | -0.60 | 0.98 |
| 5 (8) | 2.8±1.28 | 76 | -0.90 | 0.99 |
| 6 (8) | 1.5±1.85* | 41 | -2.20 | 0.98 |
| 7 (8) | 1.1±1.09** | 30 | -2.60 | 0.95 |

[1]  Each group received treatments as indicated in Table I.

[2]  Change in tumor size (Day 6-Day 0) in ocular micrometer units (omu).

[3]  Δ tumor size (test) minus Δ tumor size (control) indicates degree of tumor inhibition in omu.

[4]  Final Body Weight/Initial Body Weight
Significance of difference from control: * $p < 0.05$;
** $p < 0.01$; *** $p < 0.001$.

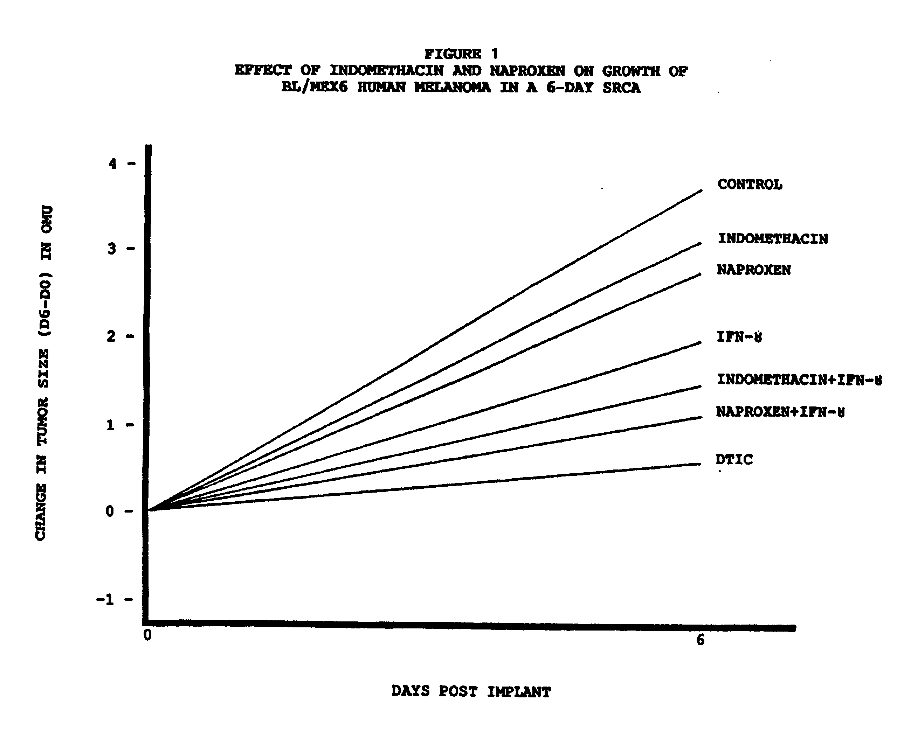

These results, which are graphically represented in Figure 1, demonstrate the effect of a combination of indomethacin and IFN-γ or naproxen and IFN-γ in inhibiting tumor growth as compared to the vehicle treated control. We observed that the inhibition of tumor growth was greater for the combination treatments than for treatments with either IFN-γ alone or indomethacin or naproxen alone. The positive chemotherapy control (DTIC) also inhibited tumor growth.

### Example 2

To demonstrate the effect of acetyl salicylic acid (ASA or aspirin) and acetaminophen on antitumor activity of IFN-γ, we treated groups 1-7 as follows :

## TABLE III

| Group # | Treatment |
|---------|-----------|
| 1 | PBS, 0.25 ml/inj., QD1-5, b.i.d., i.m. (vehicle treated control) |
| 2 | DTIC, 400 mg/Kg/inj., QD1-5, s.c. (positive chemotherapy control) |
| 3 | IFN-$\gamma$, 2000 $\mu$g/Kg/inj., QD1-5, b.i.d., i.m. |
| 4 | ASA, 328.0 mg/Kg/inj., QD1-5, b.i.d., p.o. |
| 5 | Acetaminophen, 192.0 mg/Kg/inj., QD1-5, b.i.d., p.o. |
| 6 | ASA, 328.0 mg/Kg/inj., QD1-5, b.i.d., p.o.; IFN-$\gamma$, 2000 $\mu$g/Kg/inj., QD1-5, b.i.d., i.m. |
| 7 | Acetaminophen, 192.0 mg/Kg/inj., QD1-5, b.i.d., p.o.; IFN-$\gamma$, 2000 $\mu$g/Kg/inj., QD1-5, b.i.d., i.m. |

The results obtained are set forth in Table IV :

## TABLE IV

### RESPONSE OF BL/MEX6 HUMAN MELANOMA TO
### ACETYL SALICYLIC ACID (ASA), ACETAMINOPHEN AND
### GAMMA INTERFERON USING THE SIX DAY SUBRENAL CAPSULE™ ASSAY

| Group No.[1] (No. Animals) | Avg. Δ Tumor Size[2] (mean ± S.D.) | Percent Test/Control[3] | Test-Control[4] | FBW/IBW[5] |
|---|---|---|---|---|
| 1 (10) | 4.0±1.17 | - | - | 0.96 |
| 2 (8) | -0.2±0.70*** | R2 | -4.20 | 0.81 |
| 3 (8) | 2.0±1.31** | 50 | -2.00 | 0.98 |
| 4 (7) | 3.2±1.95 | 80 | -0.80 | 0.93 |
| 5 (8) | 1.9±1.46** | 48 | -2.10 | 0.96 |
| 6 (7) | 0.6±0.85*** | 15 | -3.40 | 0.94 |
| 7 (7) | 1.6±1.27*** | 40 | -2.70 | 0.96 |

[1]  Each group was treated as indicated in Table III.

[2]  Change in tumor size (Day 6-Day 0) in ocular micrometer units (omu).

[3]  R = regression from original size using the formula FTS (final tumor size)-ITS (initial tumor size)/ITS x 100.

[4]  Δ tumor size (test) minus Δ tumor size (control) indicates degree of tumor inhibition in omu.

[5]  Final Body Weight/Initial Body Weight
Significance of difference from control: * $p < 0.05$; ** $p < 0.01$; *** $p < 0.001$.

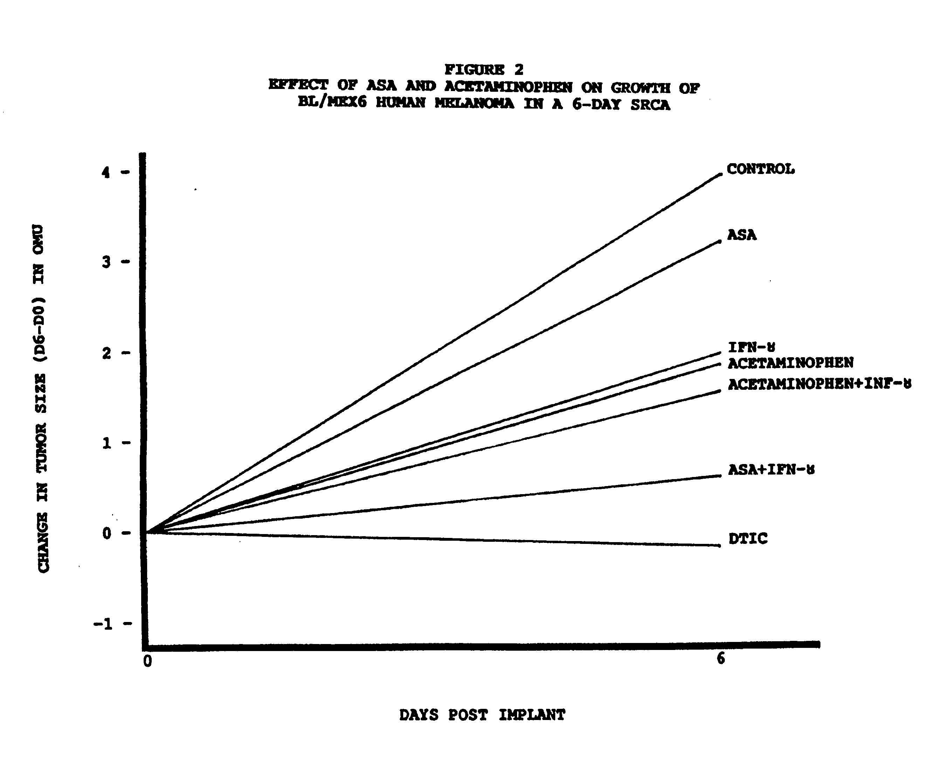

These results, which are graphically represented in Figure 2, demonstrate the effects of a combination of acetaminophen and IFN-γ or a combination of acetyl salicylic acid (ASA) and IFN-γ in inhibiting tumor growth as compared to the vehicle treated control. We observed that the combination treatments resulted in greater inhibition of tumor growth than treatment with either IFN-γ alone or acetaminophen or acetyl salicylic acid alone. The positive chemotherapy control (DTIC) also inhibited tumor growth.

### Example 3

To demonstrate the effect of cortisone and prednisone on antitumor activity of IFN-γ, we treated groups 1-7 of mice as follows :

## TABLE V

| Group # | Treatment |
|---|---|
| 1 | PBS, 0.25 ml/inj., QD1-5, b.i.d., i.m. (vehicle treated control) |
| 2 | DTIC, 400 mg/Kg/inj., QD1-5, s.c. (positive chemotherapy control) |
| 3 | IFN-$\gamma$, 2000 $\mu$g/Kg/inj., QD1-5, b.i.d., i.m. |
| 4 | Cortisone, 20.0 mg/Kg/inj., QD1-5, b.i.d., s.c. |
| 5 | Prednisone, 20.0 mg/Kg/inj., QD1-5, b.i.d., s.c. |
| 6 | Cortisone, 20.0 mg/Kg/inj., QD1-5, b.i.d., s.c.; IFN-$\gamma$, 2000 $\mu$g/Kg/inj., QD1-5, b.i.d., i.m. |
| 7 | Prednisone, 20.0 mg/Kg/inj., QD1-5, b.i.d., s.c.; IFN-$\gamma$, 2000 $\mu$g/Kg/inj., QD1-5, b.i.d., i.m. |

The results obtained are set forth in Table VI :

## TABLE VI

### RESPONSE OF BL/MEX6 HUMAN MELANOMA TO CORTISONE, PREDNISONE AND GAMMA INTERFERON USING THE SIX DAY SUBRENAL CAPSULE™ ASSAY

| Group No.[1] (No. Animals) | Avg. $\Delta$ Tumor Size[2] (mean ± S.D.) | Percent Test/Control[3] | Test-Control[4] | FBW/IBW[5] |
|---|---|---|---|---|
| 1 (10) | 3.5±1.61 | – | – | 1.04 |
| 2 (8) | −0.2±1.00*** | R2 | −3.70 | 0.81 |
| 3 (8) | 2.1±1.09* | 60 | −1.40 | 1.00 |
| 4 (8) | 1.3±1.73* | 37 | −2.20 | 0.96 |
| 5 (8) | 0.6±1.57** | 17 | −2.90 | 0.97 |
| 6 (8) | −0.3±0.93*** | R3 | −3.80 | 0.94 |
| 7 (8) | 1.1±0.99** | 31 | −2.40 | 0.92 |

[1]    Each group received treatment as indicated in Table V.

[2]    Change in tumor size (Day 6–Day 0) in ocular micrometer units (omu).

[3]    R = regression from original size using the formula (FTS-ITS)/ITS x 100.

[4]    $\Delta$ tumor size (test) minus $\Delta$ tumor size (control) indicates degree of tumor inhibition in omu.

[5]    Final Body Weight/Initial Body Weight
   Significance of difference from control: * $p < 0.05$;
   ** $p < 0.01$; *** $p < 0.001$.

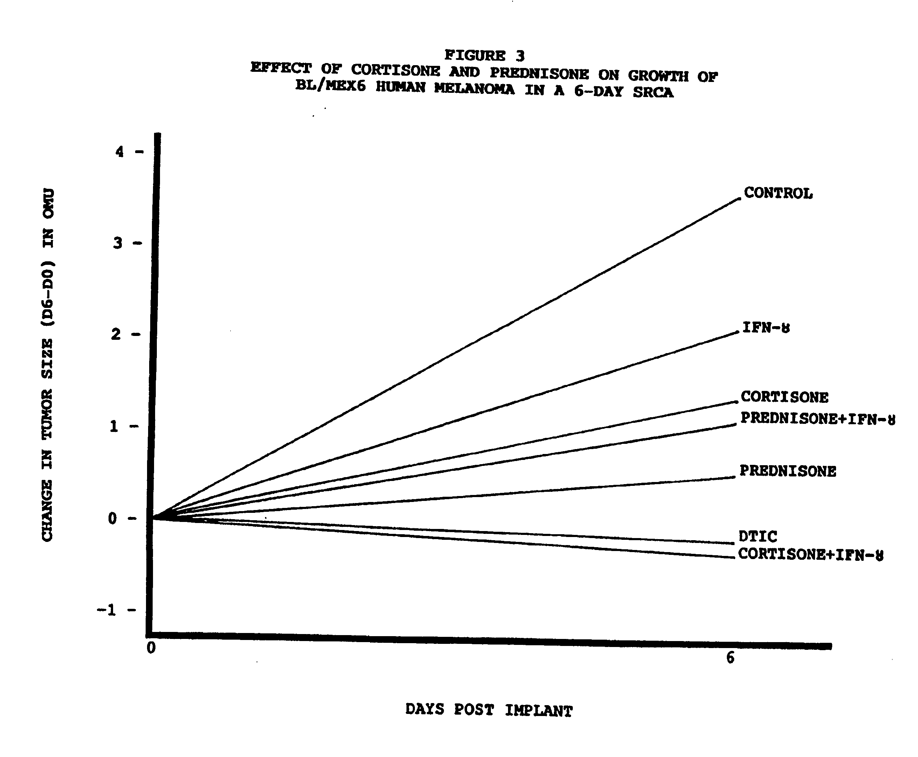

These results, which are graphically represented in Figure 3, demonstrate the effects of a combination of prednisone and IFN-γ or a combination of cortisone and IFN-γ in inhibiting tumor growth as compared to the vehicle treated control. We observed that the inhibition of tumor growth was greater for the combination treatments than for treatments with IFN-γ alone, prednisone alone or cortisone alone. The inhibition by the combination of cortisone and IFN-γ was also greater than that achieved by the DTIC positive chemotherapy control.

The following table summarizes the results obtained in Examples 1-3 :

## TABLE VII

### SUMMARY OF RESPONSE OF BL/MEX6 HUMAN MELANOMA TO ANTI-INFLAMMATORY AND ANTI-PYRETIC AGENTS IN THE 6-DAY SUBRENAL CAPSULE™ ASSAY

| Treatment* | Tumor Response |
|---|---|
| Gamma Interferon (IFN-$\gamma$) | + |
| Indomethacin | - |
| Naproxen | - |
| Indomethacin + IFN-$\gamma$ | + |
| Naproxen + IFN-$\gamma$ | ++ |
| ASA | - |
| Acetaminophen | ++ |
| ASA + IFN-$\gamma$ | +++ |
| Acetaminophen + IFN-$\gamma$ | +++ |
| Cortisone | + |
| Prednisone | ++ |
| Cortisone + IFN-$\gamma$ | +++ |
| Prednisone + IFN-$\gamma$ | ++ |

* IFN-$\gamma$ administered i.m., b.i.d., QD1-5; cortisone and prednisone administered s.c., b.i.d., QD1-5; and all other agents p.o., b.i.d., QD1-5.

| | |
|---|---|
| - | No significant inhibition |
| + | Significant inhibition $p < 0.05$ |
| ++ | Significant inhibition $p < 0.01$ |
| +++ | Significant inhibition $p < 0.001$ |

## Claims

1. A combination of IFN-$\gamma$ and a compound effective to enhance the immunotherapeutic activity of IFN-$\gamma$ selected from steroidal anti-inflammatory agents, non-steroidal anti-inflammatory agents and anti-pyretic agents.

2. The combination according to claim 1, wherein the IFN-$\gamma$ is selected from natural IFN-$\gamma$, recombinant IFN-$\gamma$, and derivatives thereof which are characterized by the immunotherapeutic activity of IFN-$\gamma$ against malignant diseases, non-malignant cell proliferative diseases, rheumatic diseases and allergic diseases.

3. The combination according to claim 1, wherein the amount of IFN-$\gamma$ is between 100 $\mu g/m^2$/day and 5 mg/m$^2$/day.

4. The combination according to claim 1, wherein the amount of IFN-γ is between 10 μg/day and 500 μg/day.

5. The combination according to claim 1, wherein the steroidal anti-inflammatory agent is selected from cortisol, prednisone, prednisolone, 6 α-methylprednisolone, cortisone, triamcinolone, paramethasone, betamethasone and dexamethasone.

6. The combination according to claim 1, wherein the non-steroidal anti-inflammatory agent is selected from acetyl salicylic acid, methyl salicylate, sodium salicylate, phenylbutazone, oxyphenbutazone, antipyrine, aminopyrine, dipyrone, apazone, indomethacin, sulindac, mefenamic acid, tolmetin sodium, ibuprofen, naproxen, fenoprofen, flurbiprofen, and ketoprofen.

7. The combination according to claim 1, wherein the anti-pyretic agent is selected from acetyl salicylic acid, methyl salicylate, sodium salicylate, phenacetin, acetaminophen, indomethacin, sulindac, mefenamic acid, tolmetin sodium, ibuprofen, naproxen, fenoprofen, flurbiprofen, and ketoprofen.

8. The combination according to claim 2, wherein the IFN-γ is recombinant IFN-γ.

9. The use of a pharmaceutically effective amount of IFN-γ and an amount of a compound effective to enhance the immunotherapeutic activity of IFN-γ selected from steroidal anti-inflammatory agents, non-steroidal anti-inflammatory agents and anti-pyretic agents for the preparation of a combination which is pharmaceutically effective to reduce the symptoms of malignant diseases, non-malignant cell proliferative diseases, rheumatic diseases and allergic diseases.

10. The use according to claim 9, wherein the IFN-γ is selected from natural IFN-γ, recombinant IFN-γ, and derivatives thereof which are characterized by the immunotherapeutic activity of IFN-γ against malignant diseases, non-malignant cell proliferative diseases, allergic diseases and rheumatic diseases.

11. The use according to claim 9, wherein the malignant disease to be treated is selected from malignant tumorigenic or neoplastic cell growth, malignant hematological systemic diseases, carcinomas, sarcomas, myelomas, melanomas, lymphomas and papillomas.

12. The use according to claim 11, wherein the pharmaceutically effective amount of IFN-γ is a dosage of between about 100 μg/m²/day and 5 mg/m²/day.

13. The use according to claim 9, wherein the disease to be treated is an allergic disease, a non-malignant cell proliferative disease, or a rheumatic disease selected from inflammatory rheumatism, degenerative rheumatism, extra-articular rheumatism, collagen diseases and asthma.

14. The use according to claim 13, wherein the pharmaceutically effective amount of IFN-γ is a dosage of between about 10 μg/day and 500 μg/day.

15. The use according to claim 9, wherein the steroidal anti-inflammatory agent is selected from cortisol, prednisone, prednisolone, 6 α-methylprednisolone, cortisone, triamcinolone, paramethasone, betamethasone and dexamethasone.

16. The use according to claim 9, wherein the non-steroidal anti-inflammatory agent is selected from acetyl salicylic acid, methyl salicylate, sodium salicylate, phenylbutazone, oxyphenbutazone, antipyrine, aminopyrine, dipyrone, apazone, indomethacin, sulindac, mefenamic acid, tolmetin sodium, ibuprofen, naproxen, fenoprofen, flurbiprofen, and ketoprofen.

17. The use according to claim 9, wherein the anti-pyretic agent is selected from acetyl salicylic acid, methyl salicylate, sodium salicylate, phenacetin, acetaminophen, indomethacin, sulindac, mefenamic acid, tolmetin sodium, ibuprofen, naproxen, fenoprofen, flurbiprofen, and ketoprofen.

18. The use according to claim 10, wherein the IFN-γ is recombinant IFN-γ.


## Patentansprüche

1. Kombination von IFN-γ und einem Stoff, der die immuntherapeutische Wirkung von IFN-γ wirksam erhöht und ein steroider entzündungshemmender Wirkstoff, ein nicht-steroider entzündungshemmender Wirkstoff oder ein fiebersenkender Wirkstoff ist.

2. Kombination nach Anspruch 1, wobei das IFN-γ natürliches IFN-γ, rekombinantes IFN-γ oder ein Derivat davon ist, das durch die immuntherapeutische Aktivität von IFN-γ gegen bösartige Krankheiten, nicht-bösartige zellproliferative Krankheiten, rheumatische Krankheiten und allergische Krankheiten gekennzeichnet ist.

3. Kombination nach Anspruch 1, wobei die Menge an IFN-γ zwischen 100 μg/m²/Tag und 5 mg/m²/Tag beträgt.

4. Kombination nach Anspruch 1, wobei die Menge an IFN-γ zwischen 10 μg/Tag und 500 μg/Tag beträgt.

5. Kombination nach Anspruch 1, wobei der steroide entzündungshemmende Wirkstoff Cortisol, Prednison, Prednisolon, 6-α-Methylprednisolon, Cortison, Triamcinolon, Paramethason, Betamethason oder Dexamethason ist.

6. Kombination nach Anspruch 1, wobei der nicht-steroide entzündungshemmende Wirkstoff Acetylsalicylsäure, Methylsalicylat, Natriumsalicylat, Phenylbutazon, Oxyphenbutazon, Antipyrin, Pyramidon, Dipyron, Azapropazon, Indometacin, Sulindac, Mefenaminsäure, Natriumtolmetin, Ibuprofen, Naproxen, Fenoprofen, Flurbiprofen oder Ketoprofen ist.

7. Kombination nach Anspruch 1, wobei der fiebersenkende Wirkstoff Acetylsalicylsäure, Methylsalicylat, Natriumsalicylat, Phenacetin, Acetaminophen (4-hydroxyacetanilid), Indometacin, Sulindac, Mefenaminsäure, Natriumtolmetin, Ibuprofen, Naproxen, Fenoprofen, Flurbiprofen oder Ketoprofen ist.

8. Kombination nach Anspruch 2, wobei das IFN-γ rekombinantes IFN-γ ist.

9. Verwendung einer pharmazeutisch wirksamen Menge an IFN-γ und einer Menge eines Stoffes, der die immuntherapeutische Wirkung von IFN-γ wirksam erhöht und ein steroider entzündungshemmender Wirkstoff, ein nicht-steroider entzündungshemmender Wirkstoff oder ein fiebersenkender Wirkstoff zur Herstellung einer Kombination, die pharmazeutisch wirksam bei der Verminderung der Symptome bösartiger Krankheiten, nicht-bösartiger Zell-proliferativer Krankheiten, rheumatischer Krankheiten und allergischer Krankheiten ist.

10. Verwendung nach Anspruch 9, wobei das IFN-γ natürliches IFN-γ rekombinantes IFN-γ oder ein Derivat davon ist, das durch die immuntherapeutische Aktivität von IFN-γ gegen bösartige Krankheiten, nicht-bösartige Zell-proliferative Krankheithen allergische Krankheiten und rheumatische Krankheiten gekennzeichnet ist.

11. Verwendung nach Anspruch 9, wobei die zu behandelnde bösartige Krankheit bösartiges tumorbildendes oder neoplastisches Zellwachstum, eine bosärtige hämatologische systemische Krankheit, ein Carcinom, Sarcom, Myelom, Melanom, Lymphom oder Papillom ist.

12. Verwendung nach Anspruch 11, wobei die pharmazeutisch wirksame Menge an IFN-γ eine Dosis von zwischen etwa 100 µg/m²/Tag und 5 mg/m²/Tag beträgt.

13. Verwendung nach Anspruch 9, wobei die zu behandelnde Krankheit eine allergische Krankheit, eine nicht-bösartige Zell-proliferative Krankheit oder eine rheumatische Krankheit, ausgewählt aus entzündlichem Rheumatismus, degenerativem Rheumatismus, extra-artikulärem Rheumatismus, Collagen-Krankheiten und Asthma ist.

14. Verwendung nach Anspruch 13, wobei die pharmazeutisch wirksame Menge an IFN-γ eine Dosis zwischen etwa 10 µg/Tag und 500 µg/Tag ist.

15. Verwendung nach Anspruch 9, wobei der steroide entzündungshemmende Wirkstoff Cortisol, Prednison, Prednisolon, 6-α-Methylprednisolon, Cortison, Triamcinolon, Paramethason, Betamethason oder Dexamethason ist.

16. Verwendung nach Anspruch 9, wobei der nicht-steroide entzündungshemmende Wirkstoff Acetylsalicylsäure, Methylsalicylat, Natriumsalicylat, Phenylbutazon, Oxyphenbutazon, Antipyrin, Pyramidon, Dipyron, Azapropazon, Indometacin, Sulindac, Mefenaminsäure, Natriumtolmetin, Ibuprofen, Naproxen, Fenoprofen, Flurbiprofen oder Ketoprofen ist.

17. Verwendung nach Anspruch 9, wobei der fiebersenkende Wirstoff Acetylsalicylsäure, Methylsalicylat, Natriumsalicylat, Phenacetin, Acetaminophen (4-hydroxyacetanilid), Indometacin, Sulindac, Mefenaminsäure, Natriumtolmetin, Ibuprofen, Naproxen, Fenoprofen, Flurbiprofen oder Keteprofen ist.

18. Verwendung nach Anspruch 10, wobei das IFN-γ rekombinantes IFN-γ ist.


## Revendications

1. Combinaison d'IFN-γ et d'un composé efficace pour augmenter l'activité immunothérapeutique d'un IFN-γ choisi parmi des agents anti-inflammatoires stéroïdiens, des agents anti-inflammatoires non stéroïdiens et des agents antipyrétiques.

2. Combinaison selon la revendication 1, dans laquelle l'IFN-γ est choisi parmi l'IFN-γ naturel, l'IFN-γ recombinant et des dérivés de ceux-ci, qui sont caractérisés par l'activité immunothérapeutique de l'IFN-γ contre les maladies malignes, les maladies prolifératives des cellules non malignes, les maladies rhumatismales et les maladies allergiques.

3. Combinaison selon la revendication 1, dans laquelle la quantité d'IFN-γ est comprise entre 100 µg/m²/jour et 5 mg/m²/jour.

4. Combinaison selon la revendication 1, dans laquelle la quantité d'IFN-γ est comprise entre 10 µg/jour et 500 µg/jour.

5. Combinaison selon la revendication 1, dans laquelle l'agent anti-inflammatoire stéroïdien est choisi parmi le cortisol, la prednisone, la prednisolone, la 6,α-méthylprednisolone, la cortisone, la triamcinolone, la paraméthasone, la bétaméthasone et la dexaméthasone.

6. Combinaison selon la revendication 1, dans laquelle l'agent anti-inflammatoire non stéroïdien est choisi parmi l'acide acétylsalicylique, le salicylate de méthyle, le salicylate de sodium, la phénylbutazone, l'oxyphenbutazone, l'antipyrine, l'aminopyrine, la dipyrone, l'apazone, l'indométhacine, le sulindac, l'acide méfénamique, la tolméthine sodique, l'ibuprofène, la naproxène, le fénoprofène, le flurbiprofène et le kétoprofène.

7. Combinaison selon la revendication 1, dans laquelle l'agent antipyrétique est choisi parmi l'acide acétylsalicylique, le salicylate de méthyle, le salicylate de sodium, la phénacétine, l'acétaminophène, l'indométhacine, le sulindac, l'acide méfénamique, la tolmétine sodique, l'ibuprofène, le naproxène, le fénoprofène, le flurbiprofène et le kétoprofène.

8. Combinaison selon la revendication 2, dans laquelle l'IFN-γ est l'IFN-γ recombinant.

9. Utilisation d'une quantité pharmaceutiquement efficace d'IFN-γ et d'une quantité d'un composé efficace pour améliorer l'activité immunothérapeutique de l'IFN-γ choisi parmi des agents anti-inflammatoires stéroïdiens, des agents anti-inflammatoires non-stéroïdiens, et des agents antipyrétiques pour la préparation d'une combinaison qui est pharmaceutiquement efficace pour réduire les symptômes de maladies malignes, de maladies prolifératives des cellules non malignes, de maladies rhumatismales et de maladies allergiques.

10. Utilisation selon la revendication 9, dans laquelle l'IFN-γ est choisi parmi l'IFN-γ naturel, l'IFN-γ recombinant et des dérivés de ceux-ci, qui sont caractérisés par l'activité immunothérapeutique de l'IFN-γ contre les maladies malignes, les maladies prolifératives des cellules non-malignes, les maladies allergiques et les maladies rhumatismales.

11. Utilisation selon la revendication 9, dans laquelle la maladie maligne à traiter est choisie parmi un développement cellulaire tumorigène ou néoplasique malin, des maladies hématologiques systémiques malignes, des carcinomes, des sarcomes, des myélomes, des mélanomes, des lymphomes et des papillomes.

12. Utilisation selon la revendication 11 dans laquelle la quantité pharmaceutiquement efficace d'IFN-γ est une dose comprise entre environ 100 μg/m²/jour et 5 mg/m²/jour.

13. Utilisation selon la revendication 9, dans laquelle la maladie à traiter est une maladie allergique, une maladie proliférative de cellules non malignes ou une maladie rhumatismale choisie parmi le rhumatisme inflammatoire, le rhumatisme dégénératif, le rhumatisme extra-articulaire, les maladies du collagène et l'asthme.

14. Utilisation selon la revendication 13, dans laquelle la quantité pharmaceutiquement efficace d'IFN-γ est une dose comprise entre environ 10 μg/jour et 500 μg/jour.

15. Utilisation selon la revendication 9, dans laquelle l'agent anti-inflammatoire stéroïdien est choisi parmi le cortisol, la prednisone, la prednisolone, la 6,α-méthylprednisolone, la cortisone, la triamcinolone, la paraméthasone, la bétaméthasone et la dexaméthasone

16. Utilisation selon la revendication 9, dans laquelle l'agent anti-inflammatoire non stéroïdien est choisi parmi l'acide acétylsalicylique, le salicylate de méthyle, le salicylate de sodium, la phénylbutazone, l'oxyphenbutazone, l'antipyrine, l'aminopyrine, la dipyrone, l'apazone, l'indométhacine, le sulindac, l'acide méfénamique, la tolméthine sodique, l'ibuprofène, la naproxène, le fénoprofène, le flurbiprofène et le kétoprofène.

17. Utilisation selon la revendication 9, dans laquelle l'agent antipyrétique est choisi parmi l'acide acétylsalicylique, le salicylate de méthyle, le salicylate de sodium, la phénacétine, l'acétaminophène, l'indométhacine, le sulindac, l'acide méfénamique, la tolméthine sodique, l'ibuprofène, le naproxène, le fénoprofène, le flurbiprofène et le kétoprofène.

18. Utilisation selon la revendication 10, dans laquelle l'IFN-γ est de l'IFN-γ recombinant.

FIGURE 1
EFFECT OF INDOMETHACIN AND NAPROXEN ON GROWTH OF
BL/MEX6 HUMAN MELANOMA IN A 6-DAY SRCA

DAYS POST IMPLANT

FIGURE 2
EFFECT OF ASA AND ACETAMINOPHEN ON GROWTH OF
BL/MEX6 HUMAN MELANOMA IN A 6-DAY SRCA

FIGURE 3
EFFECT OF CORTISONE AND PREDNISONE ON GROWTH OF
BL/MEX6 HUMAN MELANOMA IN A 6-DAY SRCA

DAYS POST IMPLANT

EP 0 311 616 B1